(19) Europäisches Patentamt
European Patent Office
Office européen des brevets

(11) **EP 2 010 896 B1**

(12) **EUROPÄISCHE PATENTSCHRIFT**

(45) Veröffentlichungstag und Bekanntmachung des Hinweises auf die Patenterteilung:
**24.10.2018 Patentblatt 2018/43**

(21) Anmeldenummer: **07722211.5**

(22) Anmeldetag: **18.04.2007**

(51) Int Cl.:
**G01N 27/07** *(2006.01)* **G01N 33/28** *(2006.01)*

(86) Internationale Anmeldenummer:
**PCT/DE2007/000653**

(87) Internationale Veröffentlichungsnummer:
**WO 2007/121708 (01.11.2007 Gazette 2007/44)**

(54) **GITTERSENSOR ZUR ZWEIDIMENSIONALEN MESSUNG VON VERSCHIEDENEN KOMPONENTEN IM QUERSCHNITT EINER MEHRPHASENSTRÖMUNG**

GRID SENSOR FOR THE TWO-DIMENSIONAL MEASUREMENT OF DIFFERENT COMPONENTS IN THE CROSS SECTION OF A MULTIPHASE FLOW

DISPOSITIF POUR LA MESURE BIDIMENSIONNELLE DE DIFFÉRENTS COMPOSANTS DANS UNE SECTION TRANSVERSALE D'UN ÉCOULEMENT MULTIPHASE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **21.04.2006 DE 102006019178**

(43) Veröffentlichungstag der Anmeldung:
**07.01.2009 Patentblatt 2009/02**

(73) Patentinhaber: **Helmholtz-Zentrum Dresden - Rossendorf e.V.**
**01328 Dresden (DE)**

(72) Erfinder:
• **DA SILVA, Marco, José**
**82510-220 Curitiba-PR (BR)**
• **SCHLEICHER, Eckhard**
**01219 Dresden (DE)**

• **HAMPEL, Uwe**
**01454 Radeberg (DE)**
• **PRASSER, Horst-Michael**
**CH-5415 Nussbaumen (CH)**

(56) Entgegenhaltungen:
DE-A1- 19 649 011  US-A- 4 644 263
US-A- 5 210 499  US-A- 5 287 752
US-B1- 6 314 373

• **H-M PRASSER A BÖTTGER ET AL: "A new electrode-mesh tomograph for gas-liquid flows" FLOW MEASUREMENT AND INSTRUMENTATION, BUTTERWORTH-HEINEMANN, OXFORD, GB, Bd. 9, 1998, Seiten 111-119, XP002389213 ISSN: 0955-5986**

EP 2 010 896 B1

**Beschreibung**

Die Erfindung betrifft eine Messanordnung zur Untersuchung von Mehrphasen- bzw. Mehrkomponentenströmungen. Typische Anwendungsgebiete sind die Analyse von Mehrphasenströmungen in der Chemieverfahrenstechnik sowie der Mineralölförderung und -verarbeitung.

In dem Artikel "A new electrode-mesh tomograph for gas-liquid flows" (H.-M. Prasser et al., Flow Measurement and Instrumentation 9, 1998, S. 111 ff.) sowie in US 4,644,263 A, US 5,210,499 A und DE 19 649 011 A1 werden gitterförmige Anordnungen beschrieben, die zur Untersuchung zweiphasiger Medien mit Hilfe einer zweidimensionalen Leitfähigkeitsmessung verwendet werden können. Bei diesen Anordnungen werden die drahtförmigen Elektroden der einen Elektrodenebene nacheinander mit Gleichspannungs- oder Bipolar-Rechteckspannungssignalen beaufschlagt und gleichzeitig wird an den Elektroden der anderen Elektrodenebene zeitgleich ein Stromsignal erfasst. Dadurch sind diese Anordnungen in der Lage die Leitfähigkeit in den Kreuzungspunkten der Elektroden zu bestimmen. Für eine Zweiphasenströmung mit genau einer leitfähigen Phase (zum Beispiel ein Gas-WasserGemisch) kann somit die Phasenverteilung im Strömungsquerschnitt durch Aufzeichnung der Leitfähigkeitsverteilung bestimmt werden. Eine Phasendiskriminierung für ähnlich gut oder schlecht leitende Phasen oder Komponenten einer Strömung ist mit diesen Anordnungen nicht direkt möglich.

In US 5,287,752 A wird eine Anordnung zur Untersuchung einer Mehrphasenströmung beschrieben, bei der ein Paar unbewegliche parallele Platten innerhalb einer Rohrleitung angebracht wird. Die Platten haben mehrere Segmente, welche als kapazitive Sensoren dienen. Es wird beschrieben, dass mittels einer Sinusspannungsanregung ein Mehrphasengemisch mittels komplexer Admittanzmessung untersucht werden kann.

Die oben genannten Anordnungen zur Messung der Leitfähigkeit haben einen wesentlichen Nachteil. Es ist mit diesen Sensoren nicht möglich, nichtleitfähige Phasen- bzw. Komponenten einer Strömung zu unterscheiden. Mit der in US 5,287,752 A offen gelegten Anordnung ist es dagegen nicht möglich, eine Visualisierung der Phasenverteilung in einem Rohrquerschnitt zu realisieren, da das Eindringen einer Sensorplatte die Strömung stark beeinflusst und nur die Erfassung der Phasenverteilung an der Oberfläche der Sensoranordnung zulässt.

Aufgabe der vorliegenden Erfindung ist es, eine Anordnung zur schnellen Messung der Phasen- oder der Komponentenverteilung in einem Strömungsquerschnitt für Stoffgemische auch nichtleitender Art auf Basis einer Messung der komplexen elektrischen Admittanz anzugeben.

Die Aufgabe wird durch die Merkmale des Anspruchs 1 gelöst. Ausgestaltungen der Erfindung sind in den Unteransprüchen ausgeführt.

Neuartig an der Erfindung ist die Möglichkeit einer schnellen zweidimensionalen Messung sowohl des elektrischen Leitwertes (oder der Leitfähigkeit) als auch der elektrischen Kapazität (oder Permittivität) des Stoffgemisches.

Mit Hilfe dieser Anordnung sowie eines angeschlossenen elektronischen Datenverarbeitungsprozesses lassen sich Phasen- und/oder Komponentenverteilungen in einem Strömungsquerschnitt, zum Beispiel innerhalb einer Rohrleitung, eines Reaktorgefäßes oder eines anderen strömungsführenden Gefäßes, mit sehr hoher zeitlicher Auflösung erfassen, darstellen und auswerten.

Die Erfindung wird nachfolgend anhand von Ausführungsbeispielen näher erläutert. Dabei werden auch einige Varianten beschrieben.

Die dargestellten Figuren werden bei der Beschreibung des jeweiligen Ausführungsbeispiels mit bezeichnet.

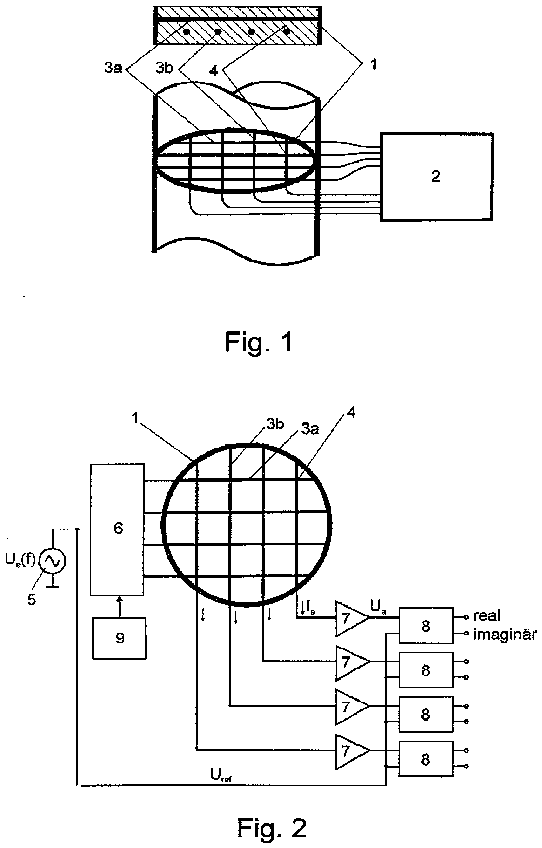

Die Anordnung gemäß Fig. 1 besteht aus einem Gittersensor (1) mit mindestens zwei in geringem Abstand zueinander befindlichen Elektrodenebenen und einer zugeordneten Messelektronik (2). Der Gittersensor besitzt zwei Ebenen drahtförmiger Elektroden, die Sendeelektroden (3a) und die Empfängerelektroden (3b), die innerhalb jeder Ebene in einem geringen Abstand parallel zueinander angeordnet sind. Die Orientierung der Elektroden verschiedener Ebenen ist etwa senkrecht zueinander. Gemessen wird die komplexwertige elektrischen Admittanz des Mediums zwischen den Sendeelektroden (3a) und den Empfängerelektroden (3b) in jedem einzelnen Kreuzungspunkt (4) des Elektrodengitters.

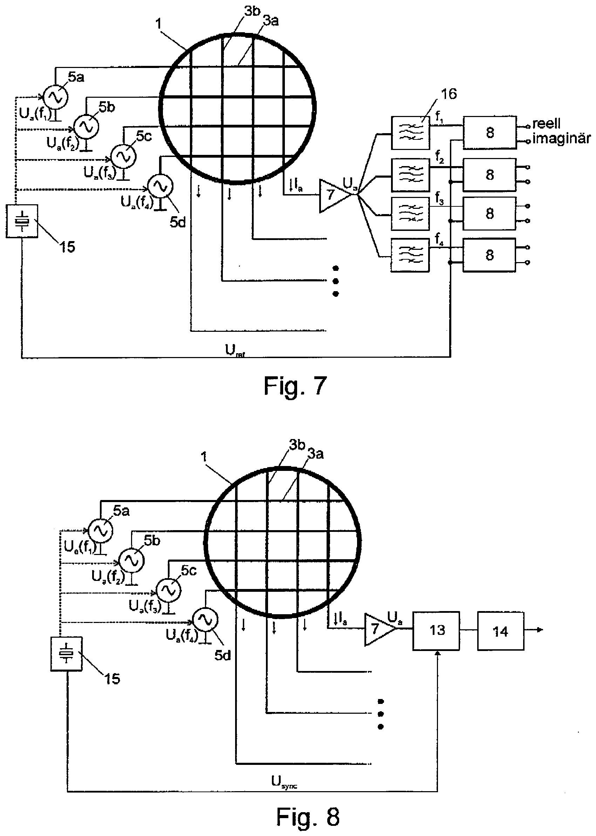

Zur Messung der komplexwertigen elektrischen Admittanz (Fig. 2) des Mediums im einzelnen Kreuzungspunkt (4) wird die zugehörige Sendeelektrode (3a) mittels eines Sinusgenerators (5) mit einer Wechselspannung beaufschlagt. Gleichzeitig wird an der Empfängerelektrode (3b) der durch das Untersuchungsmedium im Kreuzungspunkt fließende Wechselstrom mit Hilfe eines an die Elektrode angeschlossenen Strom-Spannungswandlers (7) in ein Wechselspannungssignal gewandelt. Dieses Wechselspannungssignal wird anschließend mit einem Vektorvoltmeter (8), dessen Referenzsignal vom Anregungssignal abgezweigt wird, erfasst und ausgewertet. Das Vektorvoltmeter (8) liefert den Real- und Imaginärteil des Spannungssignals, oder äquivalent dessen Betrag und Phase, relativ zum Anregungssignal des Sinusgenerators (5). Um zu einem zweidimensionalen Bild der Phasen- bzw. Komponentenverteilung in der Gitterebene zu gelangen, müssen die komplexen Admittanzen der Gitterkreuzpunkte (4) sehr schnell gleichzeitig oder nacheinander vermessen werden. Dies kann mit Hilfe eines Multiplexers (6) und eines entsprechenden Kontrollers (9) oder mittels eines Mehrfrequenz-Anregungsschemas (Fig. 7, Fig. 8) mit mehreren Sinusgeneratoren (5a, 5b, 5c und 5d) erfolgen.

Fig. 2, Fig. 5, Fig. 6, Fig. 7, Fig. 8 zeigen schematisch Gittersensoranordnungen mit 4 x 4 Elektroden und einer Rundgeometrie. Die Gittersensoren können natürlich auch in anderen Geometrien, z. B. rechteckigen Querschnitten, aufgebaut sein. Weiterhin ist die Anzahl von Elektroden theoretisch beliebig.

Fig. 1 und Fig. 2 zeigen schematisch einen Gittersensor (1) mit zwei Elektrodenebenen und eine dazugehörige Messelektronik (2). Der Gittersensor verfügt über je vier Metalldrähte pro Ebene (3a, 3b), welche elektrisch voneinander isoliert über den Sensorquerschnitt gespannt sind. Die Verankerung der Drähte im Sensorrahmen erfolgt derartig, dass jede Elektrode vollständig von den anderen Elektroden sowie dem Rahmen selbst elektrisch isoliert ist. Die Messelektronik besteht auf der Anregungsseite aus einem Sinusgenerator (5), einem Multiplexer (6) und einem Kontroller (9). Die einzelnen Sendeelektroden (3a) der Sendeebene des Sensors sind elektrisch mit den Ausgängen des Multiplexers (6) verbunden. Auf der Empfängerseite ist jede der Empfängerelektroden (3b) der Empfängerebene mit einem Strom-Spannungswandler (7) verbunden. An die Strom-Spannungswandler (7) sind zur Auswertung des Kompleximpedanzsignals Vektorvoltmeter (8) angeschlossen, deren Referenzeingänge mit dem Signalausgang des Sinusgenerators (5) verbunden sind.

Das Messschema des in Fig. 1 skizzierten Sensors ist wie folgt:

Durch einen für die Steuerung vorgesehenen Kontroller oder Mikroprozessor (9) wird das Sinusspannungssignal des Sinusgenerators (5) nacheinander auf die einzelnen Sendeelektroden (3a) über den Multiplexer (6) aufgeschaltet. Der Multiplexer (6) ist so ausgeführt, dass nur eine einzelne Sendeelektrode (3a) mit der Sinusspannung beaufschlagt wird, während alle anderen Sendeelektroden auf Nullpotential liegen. An der jeweils aktiven Sendeelektrode fließt in den Kreuzungspunkten (4) der Drahtelektroden ein Wechselstrom zu den auf virtueller Masse liegenden Empfängerelektroden (3b). Dieser Strom ist proportional zu der aktuellen Admittanz Y des Kreuzungspunkts und wird durch die Strom-Spannungswandler (7) in ein Spannungssignal $U_a$ gewandelt. Es gilt $U_a=k \cdot I_a$, wobei $I_a$ den Wechselstrom im Kreuzungspunkt und k die Transimpedanz-Verstärkung des Strom-Spannungswandlers bezeichnet. Durch die Vektorvoltmeter (8) wird das Wechselspannungssignal $U_a$ mit dem Anregungseingangssignal $U_e$ verglichen und nach Betrag und Phase ausgewertet.

Nach dem Ohmschen Gesetz gilt für die Admittanz

$$Y = \frac{I_a}{U_e} = \frac{U_a}{kU_e} \,.$$

Diese kann aus dem resultierenden Phasenzeiger $U_a/U_e$ direkt berechnet werden.

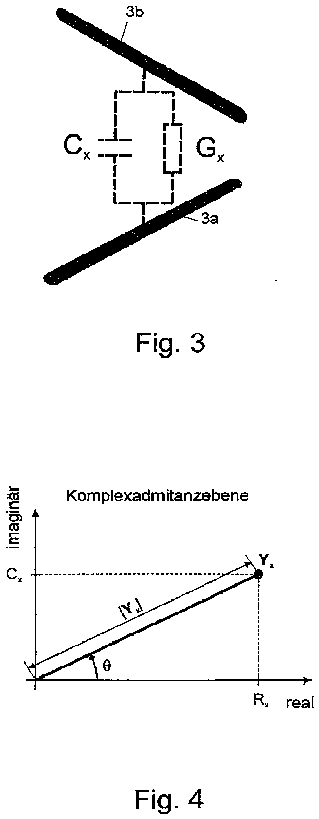

Fig. 4 verdeutlicht die Zusammenhänge der Admittanz in der komplexen Ebene.

Das Ersatzschaltbild für einen Kreuzungspunkt (4) und die sich daraus ergebenden Messgrößen sind in Fig. 3 dargestellt. Dabei bedeuten (3a) Sendeelektrode und (3b) Empfängerelektrode. Die elektrische Admittanz ist mit dem Leitwert $G_x$ und der Kapazität $C_x$ des Mediums am Kreuzungspunkt gemäß

$$Y_x = G_x + j \cdot 2\pi f \cdot C_x$$

verknüpft, wobei $j = \sqrt{-1}$ und f die Anregungsfrequenz des Sinusgenerators (5) ist.

Schaltungstechnisch sind folgende Varianten zur Realisierung des Vektorvoltmeters (8) denkbar:

1. Realisierung als Amplituden-Phasen-Detektor, wobei der Betrag $|Y_x|$ und die Phase $\theta$ des komplexen Ausgangsspannungsverhältnisses $U_a/U_e$ bestimmt werden. Die Messgrößen $G_x$ und $C_x$ können dementsprechend aus:

$$G_x = |Y_x|\cos\theta$$

$$C_x = \frac{|Y_x|\cos\theta}{2\pi f}$$

berechnet werden.

2. Realisierung als I/Q-Demodulator, wobei die reelle und die imaginäre Komponente des komplexen Ausgangsspannungsverhältnisses $U_a/U_e$ bestimmt werden. Die gesuchten Messgrößen ergeben sich dann aus:

$$G_x = Re(Y_x)$$

$$C_x = \frac{Im(Y_x)}{2\pi f}.$$

3. Realisierung durch eine hinreichend schnelle zeitliche Abtastung und digitale Aufzeichnung der Spannungssignale $U_a$ und $U_e$ bzw. deren Verhältnisses mit Hilfe eines Analog-Digital-Wandlers und anschließende Software- oder hardwarebasierte Fourieranalyse.

In der Materialwissenschaft und in der analytischen Chemie ist geläufig, Materialien durch ihre komplexe relative Permittivität $\varepsilon_r{}^*$ zu kennzeichnen. Diese komplexe Größe beinhaltet die Dielektrizitätszahl $\varepsilon_r$ und die Leitfähigkeit $\kappa$

$$\varepsilon_r{}^* = \varepsilon_r - j\frac{\kappa}{2\pi f \cdot \varepsilon_0}.$$

Die komplexe Admittanz ist direkt proportional zu der komplexen relativen Permittivität

$$Y = \varepsilon_r{}^* \cdot \varepsilon_0 \cdot j2\pi f \cdot k_g,$$

wobei $k_g$ ein Geometriefaktor der Messanordnung und $\varepsilon_0$ die Dielektrizitätszahl von Vakuum (8,85 pF/m) bezeichnet.

Die vorliegende Anordnung ist auch in der Lage, die komplexe relative Permittivität $\varepsilon_r{}^*$ zu bestimmen. Dem Fachmann ist geläufig, dass dafür eine Kalibrierung mit einem bekannten Medium, wie etwa Wasser oder Luft, benötigt wird. Aus dieser Kalibrierung wird der Geometriefaktor $k_g$ bestimmt. Folglich kann aus der Admittanzmessung die komplexe relative Permittivität $\varepsilon_r{}^*$ bestimmt werden. Besteht die Zielstellung lediglich in der Stoffunterscheidung, ist jedoch keine Kalibrierung nötig, da die gemessenen Parameter $G_x$ und $C_x$ bereits die gesuchte Information tragen und direkt ausgewertet werden können.

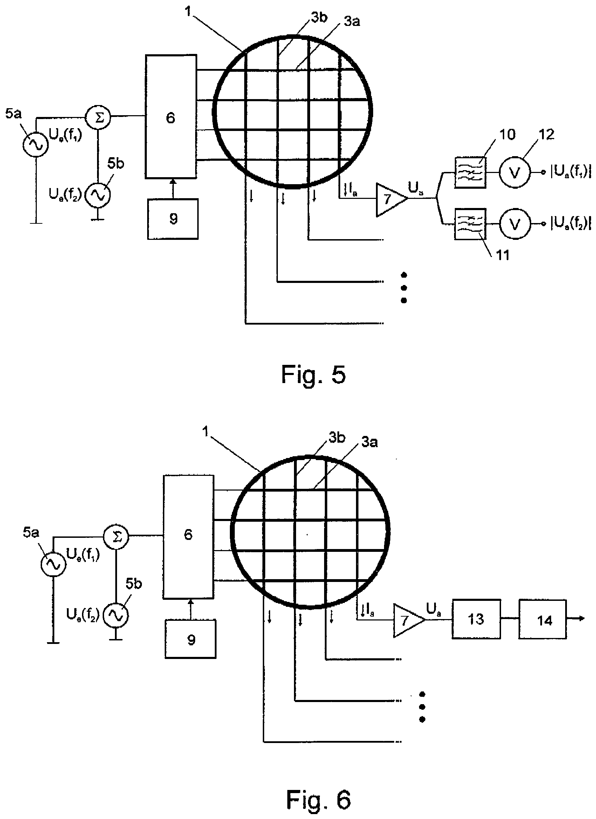

Die Zeichnung Fig. 5 zeigt eine weitere schaltungstechnische Variante zur Bestimmung der beiden Messgrößen $G_x$ und $C_x$. Anstatt die Sendeelektroden (3a) mit einer Wechselspannung einer einzelnen Frequenz f zu beaufschlagen, wird ein Wechselspannungssignal mit zwei Frequenzen $f_1$ und $f_2$ mittels zweier Sinusgeneratoren (5a, 5b) oder eines Zweifrequenzsinusgenerators generiert und auf die durch den Multiplexer (6) selektierte Sendeelektrode (3a) gelegt. Die beiden Frequenzkomponenten werden am Ausgang des Strom-Spannungswandlers (7) durch eine Kombination aus Hochpassfilter (10) und Tiefpassfilter (11) getrennt und einzeln durch skalare Voltmeter (12) ausgewertet. Die Messgrößen $G_x$ und $C_x$ können dann durch die Lösung des Gleichungssystems

$$\left|Y_x(f_1)\right|^2 = G_x{}^2 + \left(j \cdot 2\pi f_1 \cdot C_x\right)^2$$

$$\left|Y_x(f_2)\right|^2 = G_x{}^2 + \left(j \cdot 2\pi f_2 \cdot C_x\right)^2$$

bestimmt werden. Somit gilt

$$C_x = \sqrt{\frac{\left|Y_x(f_1)\right|^2 - \left|Y_x(f_2)\right|^2}{\left(2\pi f_1\right)^2 - \left(2\pi f_2\right)^2}}$$

und

$$G_x = \sqrt{\frac{\left|Y_x(f_2)\right|^2 \cdot (2\pi f_1)^2 - \left|Y_x(f_1)\right|^2 \cdot (2\pi f_2)^2}{(2\pi f_1)^2 - (2\pi f_2)^2}} \; .$$

In diesem Fall reicht also die Messung der Amplituden der Wechselspannungssignale durch beispielweise Spitzen- oder Effektivwertdetektion aus. Weiterhin können durch Analog-Digital-Wandlung (13) des Zweifrequenzsignals und anschließende Fourieranalyse (14) die Beträge der einzelnen Frequenzkomponenten bestimmt werden (Fig. 6).

Bei den in Fig. 7 und Fig. 8 dargestellten Anordnungen wird schließlich ein Mehrfrequenz-Anregungsschema benutzt. Hierbei ist jede einzelne Sendeelektrode (3a) der Anregungsebene direkt mit einem Sinusgenerator (5a, 5b, 5c und 5d) verbunden. Die Sinusgeneratoren erzeugen Wechselspannungen unterschiedlicher Frequenzen, sind aber durch einen gemeinsamen Taktgeber (15) miteinander synchronisiert. Der Messstrom $I_a$ setzt sich nunmehr summativ aus den durch die komplexe Admittanz der Kreuzungspunkte gewichteten Frequenzanteilen der verschiedenen Anregungsfrequenzen $f_1$, $f_2$, $f_3$, und $f_4$ zusammen. Jeder dieser Frequenzanteile ist eindeutig einem Kreuzungspunkt (4) zugeordnet. Zur Trennung der Signalanteile sind an die Ausgänge der Strom-Spannungswandler Bandpassfilter (16) angeschlossen, die die jeweilige Frequenzkomponente selektiv durchlassen. In diesem Ausführungsbeispiel werden pro Empfängerdraht vier Bandpassfilter (16) und vier Vektorvoltmeter (8) zur Auswertung des komplexen Ausgangsspannungssignals benötigt. Dabei kann wieder jede der oben genannten Realisierungsvarianten des Vektorvoltmeters verwendet werden. Für die in Fig. 8 dargestellte Variante 3 (zeitliche Abtastung mit anschließender Fourieranalyse) ist kein Bandpassfilter erforderlich, da die Trennung der Frequenzanteile bei Fourieranalyse (14) automatisch erfolgt. Die Abtastung der Signale durch Analog-Digital-Wandler (13) muss dabei synchron mit dem Taktgeber (15) des Anregungssignals erfolgen.

Für den Fall einer reinen Leitfähigkeits- oder Kapazitätsmessung kann das Vektorvoltmeter (8) durch ein herkömmliches skalares Voltmeter (Spitzen- oder Effektivwertdetektor) ersetzt werden, denn in diesem Fall muss nur noch der Betrag des Messsignals und nicht mehr die Phasenlage ausgewertet werden. Eine reine Kapazitätsmessung kann von Vorteil sein, da die Elektroden nicht elektrisch direkt dem zu untersuchenden Medium ausgesetzt werden müssen. Damit ist es möglich, die Elektroden ausreichend vor Korrosion oder elektrochemischer Zersetzung zu schützen. Weiterhin wird die elektrische Isolation der Elektroden an den Fixierpunkten im Sensorrahmen erleichtert.

Bezugszeichenliste

1 - Gittersensor
2 - Messelektronik
3a - Sendeelektrode
3b - Empfängerelektrode
4 - Kreuzungspunkt
5 - Sinusgenerator
6 - Multiplexer
7 - Strom-Spannungswandler
8 - Vektorvoltmeter
9 - Kontroller
10 - Hochpassfilter
11 - Tiefpassfilter
12 - skalares Voltmeter
13 - Analog-Digital-Wandler
14 - Fourieranalyse
15 - Taktgeber
16 - Bandpassfilter

**Patentansprüche**

**1.** Anordnung zur zweidimensionalen Messung von verschiedenen Komponenten im Querschnitt einer Mehrphasenströmung, **bestehend aus**

- einem Gittersensor (1), dessen Gitter aus draht- oder stabförmigen Sendeelektroden (3a) und Empfängerelektroden (3b) aufgebaut ist, die in zwei koplanaren Ebenen im Abstand von wenigen Millimetern angeordnet sind, wobei die Elektroden innerhalb jeder Ebene parallel zueinander und Elektroden unterschiedlicher Ebenen unter Ausbildung von Kreuzungspunkten (4) im Winkel von etwa 90° zueinander angeordnet sind, und
- einer zugeordneten Messelektronik (2) mit Strom-Spannungswandlern (7), die den Empfängerelektroden (3b) nachgeschaltet sind und zum Verstärken eines von mindestens einer der Sendeelektroden (3a) durch die Mehrphasenströmung zu den Empfängerelektroden (3b) fließenden Wechselstromes und Umwandeln des verstärkten Wechselstromes in ein als Ausgangsspannungssignal fungierendes Wechselspannungssignal ($U_a$) ausgebildet sind,

**dadurch gekennzeichnet, dass**

- die Messelektronik (2) mindestens einen Sinusgenerator (5) zum Erzeugen eines als Eingangsspannungssignal fungierenden Wechselspannungssignals ($U_e$) aufweist, der den Sendeelektroden (3a) vorgeschaltet ist und zum Beaufschlagen der Sendeelektroden (3a) mit dem Eingangsspannungssignal ($U_e$) ausgebildet ist, und
- die Messelektronik (2) Vektorvoltmeter (8) aufweist, die den Strom-Spannungswandlern (7) nachgeschaltet sind und zum Erfassen des komplexen Signalverhältnisses ($U_a/U_e$) zwischen dem Ausgangsspannungssignal ($U_a$) und dem Eingangsspannungssignal ($U_e$) ausgebildet sind.

2. Anordnung nach Anspruch 1, **dadurch gekennzeichnet, dass** die Messelektronik (2) einen Multiplexer (6) aufweist, der den Sendeelektroden (3a) vorgeschaltet ist und derart ausgebildet ist, dass von ihm beim Betreiben der Anordnung in einem definierten Zeitregime das als Eingangsspannungssignal fungierende Wechselspannungssignal ($U_e$) des Sinusgenerators (5) nacheinander auf die Sendeelektroden aufgeschaltet wird.

3. Anordnung nach Anspruch 1 oder 2, **dadurch gekennzeichnet, dass** der mindestens eine Sinusgenerator (5) ein Zweifrequenzsinusgenerator (5a, 5b) ist, der zum Beaufschlagen der Sendeelektroden (3a) mit einem Wechselspannungssignal mit zwei Frequenzanteilen ausgebildet ist.

4. Anordnung nach Anspruch 1, **dadurch gekennzeichnet, dass** die Messelektronik (2) mehrere Sinusgeneratoren (5a, 5b, 5c, 5d) aufweist, die den Sendeelektroden (3a) vorgeschaltet sind und zum Erzeugen von Wechselspannungen unterschiedlicher Frequenzen ausgebildet sind, wobei jede der Sendeelektroden (3a) durch einen separaten der Sinusgeneratoren (5a, 5b, 5c, 5d) mit einem Wechselspannungssignal eigener Frequenz beaufschlagbar ist, und wobei die Sinusgeneratoren phasensynchron verschaltet sind.

5. Anordnung nach Anspruch 4, **dadurch gekennzeichnet, dass** jedem der Strom-Spannungswandler (7) als Bestandteil der Messelektronik (2) ein Set von Bandpassfiltern (16) und Vektorvoltmetern (8) nachgeschaltet ist, mit deren Hilfe die Analyse der von den Strom-Spannungswandlern (7) als Ausgangsspannungssignale ($U_a$) erzeugten Wechselspannungssignale für alle der unterschiedlichen Frequenzen der mehreren Sinusgeneratoren (5a, 5b, 5c, 5d) erfolgt.

6. Anordnung nach einem der Ansprüche 1 bis 5, **dadurch gekennzeichnet, dass** jedes der Vektorvoltmeter (8) einen Analog-Digital-Wandler (13) und einen Fourieranalysator (14) aufweist, wobei der Analog-Digital-Wandler (13) zum Erfassen eines der von den Strom-Spannungswandlern (7) als Ausgangsspannungssignale generierten Wechselspannungssignale ($U_a$) mit einer vorgegebenen Abtastrate ausgebildet ist, und wobei der Fourieranalysator (14) zum Durchführen einer digitalen software- oder hardwarebasierten Fourieranalyse (14) des von dem Analog-Digital-Wandler (13) abgetasteten Wechselspannungssignals ausgebildet ist.

7. Anordnung nach Anspruch 1, **dadurch gekennzeichnet, dass** die Elektroden des Gittersensors (1) vollständig von einer elektrisch isolierenden Schutzschicht umgeben sind.

**Claims**

1. Arrangement for the two-dimensional measurement of different components in the cross section of a multiphase flow, **comprising**

- a grid sensor (1), the grid of which is constructed from wire-shaped or rod-shaped transmission electrodes (3a) and receiver electrodes (3b) which are arranged at a distance of a few millimetres in two coplanar planes, wherein the electrodes within each plane are arranged parallel to one another and electrodes on different planes are arranged at an angle of approximately 90° with respect to one another so as to form intersection points (4), and
- associated measuring electronics (2) having current-voltage converters (7) which are arranged downstream of the receiver electrodes (3b) and are designed to amplify an alternating current flowing from at least one of the transmission electrodes (3a), through the multiphase flow, to the receiver electrodes (3b) and to convert the amplified alternating current into an AC voltage signal ($U_a$) acting as an output voltage signal,

**characterized in that**

- the measuring electrodes (2) have at least one sine-wave generator (5) for generating an AC voltage signal ($U_e$) acting as an input voltage signal, which sine-wave generator is connected upstream of the transmission electrodes (3a) and is designed to apply the input voltage signal ($U_e$) to the transmission electrodes (3a), and
- the measuring electronics (2) have vector voltmeters (8) which are connected downstream of the current-voltage converters (7) and are designed to capture the complex signal ratio ($U_a/U_e$) between the output voltage signal ($U_a$) and the input voltage signal ($U_e$) .

2. Arrangement according to Claim 1, **characterized in that** the measuring electronics (2) have a multiplexer (6) which is connected upstream of the transmission electrodes (3a) and is designed in such a manner that, during operation of the arrangement, it applies the AC voltage signal ($U_e$) from the sine-wave generator (5), which acts as the input voltage signal, to the transmission electrodes in succession in a defined time regime.

3. Arrangement according to Claim 1 or 2, **characterized in that** the at least one sine-wave generator (5) is a two-frequency sine-wave generator (5a, 5b) which is designed to apply an AC voltage signal having two frequency components to the transmission electrodes (3a).

4. Arrangement according to Claim 1, **characterized in that** the measuring electronics (2) have a plurality of sine-wave generators (5a, 5b, 5c, 5d) which are connected upstream of the transmission electrodes (3a) and are designed to generate AC voltages at different frequencies, wherein an AC voltage signal at a separate frequency can be applied to each of the transmission electrodes (3a) by a separate one of the sine-wave generators (5a, 5b, 5c, 5d), and wherein the sine-wave generators are connected in a phase-synchronous manner.

5. Arrangement according to Claim 4, **characterized in that** a set of bandpass filters (16) and vector voltmeters (8) is connected downstream of each of the current-voltage converters (7) as part of the measuring electronics (2) and is used to analyse the AC voltage signals generated by the current-voltage converters (7) as output voltage signals ($U_a$) for all of the different frequencies of the plurality of sine-wave generators (5a, 5b, 5c, 5d).

6. Arrangement according to one of Claims 1 to 5, **characterized in that** each of the vector voltmeters (8) has an analogue/digital converter (13) and a Fourier analyser (14), wherein the analogue/digital converter (13) is designed to capture one of the AC voltage signals ($U_a$) generated by the current-voltage converters (7) as output voltage signals at a predefined sampling rate, and wherein the Fourier analyser (14) is designed to carry out a digital software-based or hardware-based Fourier analysis (14) of the AC voltage signal sampled by the analogue/digital converter (13).

7. Arrangement according to Claim 1, **characterized in that** the electrodes of the grid sensor (1) are completely surrounded by an electrically insulating protective layer.

**Revendications**

1. Dispositif de mesure bidimensionnelle de divers composants dans la section transversale d'un écoulement multiphasique, comprenant

- un capteur à grille (1), dont la grille est constituée d'électrodes d'émission (3a) et d'électrodes de réception (3b) en forme de fil ou de tige qui sont disposées dans deux plans coplanaires espacés de quelques millimètres, les électrodes étant disposées parallèlement les unes aux autres à l'intérieur de chaque couche et des électrodes de plans différents étant disposées selon un angle d'environ 90° les unes par rapport aux autres de façon à former des points d'intersection (4), et
- une électronique de mesure associée (2) comportant des convertisseurs courant-tension (7) qui sont montés en aval des électrodes de réception (3b) et qui sont conçus pour amplifier un courant alternatif circulant depuis au moins une des électrodes d'émission (3a) vers les électrodes de réception (3b) à travers l'écoulement multiphasique et pour convertir le courant alternatif amplifié en un signal de tension alternative ($U_a$) faisant office de signal de tension de sortie, **caractérisé en ce que**

- l'électronique de mesure (2) comprend au moins un générateur d'onde sinusoïdale (5) destiné à générer un signal de tension alternative (Ue), faisant office de signal de tension d'entrée, lequel générateur d'onde sinusoïdale est monté en amont des électrodes d'émission (3a) et est conçu pour appliquer le signal de tension d'entrée ($U_e$) sur les électrodes d'émission (3a), et

- l'électronique de mesure (2) comporte des voltmètres vectoriels (8) qui sont montés en aval des convertisseurs courant-tension (7) et qui sont conçus pour détecter le rapport de signaux complexe ($U_a/U_e$) entre le signal de tension de sortie ($U_a$) et le signal de tension d'entrée ($U_e$).

2. Dispositif selon la revendication 1, **caractérisé en ce que** l'électronique de mesure (2) comprend un multiplexeur (6) qui est monté en amont des électrodes d'émission (3a) et qui est conçu de façon à commuter successivement sur les électrodes d'émission, lorsque le dispositif fonctionne dans un régime de temps défini, le signal de tension alternative ($U_e$), faisant office de signal de tension d'entrée, du générateur d'onde sinusoïdale (5).

3. Dispositif selon la revendication 1 ou 2, **caractérisé en ce que** l'au moins un générateur d'onde sinusoïdale (5) est un générateur d'onde sinusoïdale à double fréquence (5a, 5b) qui est conçu pour appliquer sur les électrodes d'émission (3a) un signal de tension alternative comportant deux composantes fréquentielles.

4. Dispositif selon la revendication 1, **caractérisé en ce que** l'électronique de mesure (2) comporte une pluralité de générateurs d'onde sinusoïdale (5a, 5b, 5c, 5d) qui sont montés en amont des électrodes d'émission (3a) et qui sont conçus pour générer des tensions alternatives de fréquences différentes, chacune des électrodes d'émission (3a) étant soumise à un signal de tension alternative de fréquence propre par un générateur séparé parmi les générateurs sinusoïdaux (5a, 5b, 5c, 5d), et les générateurs d'onde sinusoïdale étant câblés en synchronisme de phase.

5. Dispositif selon la revendication 4, **caractérisé en ce qu'**un ensemble de filtres passe-bande (16) et de voltmètres vectoriels (8) est monté en aval de chacun des convertisseurs courant-tension (7) en tant que partie de l'électronique de mesure (2), ensemble au moyen duquel l'analyse des signaux de tension alternative, générés comme signaux de tension de sortie ($U_a$) par les convertisseurs courant-tension (7), est effectuée pour toutes les différentes fréquences de la pluralité de générateurs d'onde sinusoïdale (5a, 5b, 5c, 5d).

6. Dispositif selon l'une des revendications 1 à 5, **caractérisé en ce que** chacun des voltmètres vectoriels (8) comporte un convertisseur analogique-numérique (13) et un analyseur de Fourier (14), le convertisseur analogique-numérique (13) étant conçu pour détecter l'un des signaux de tension alternative ($U_a$) générés comme signaux de tension de sortie par les convertisseurs courant-tension (7), avec une fréquence d'échantillonnage prédéterminée, et l'analyseur de Fourier (14) étant conçu pour effectuer une analyse de Fourier numérique (14), matérielle ou logicielle, du signal de tension alternative échantillonné par le convertisseur analogique-numérique (13).

7. Dispositif selon la revendication 1, **caractérisé en ce que** les électrodes du capteur à grille (1) sont entièrement entourées par une couche de protection électriquement isolante.

Fig. 1

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4644263 A
- US 5210499 A
- DE 19649011 A1
- US 5287752 A

**In der Beschreibung aufgeführte Nicht-Patentliteratur**

- **H.-M. PRASSER et al.** A new electrode-mesh tomograph for gas-liquid flows. *Flow Measurement and Instrumentation,* 1998, vol. 9, 111 ff